# EUROPÄISCHE PATENTSCHRIFT

(11) **EP 1 952 703 B1**
(45) Veröffentlichungstag und Bekanntmachung des Hinweises auf die Patenterteilung: **25.08.2010**
(21) Anmeldenummer: 07002352.8
(22) Anmeldetag: 02.02.2007
(51) Int. Cl.: A23L 1/30, B65D 51/28, A23L 1/03

(54) **Produkt mit lebenden probiotischen Mikroorganismen**
Product including living probiotic micro organisms
Produit contenant des microorganismes probiotiques vivants

(43) Veröffentlichungstag der Anmeldung: 06.08.2008
(73) Patentinhaber: May, Amadeus Alexander, 9050 Appenzell (CH)
(72) Erfinder: May, Maximilian Emanuel, 85662 Hohenbrunn (DE)
(74) Vertreter: Draudt, Jutta

(56) Entgegenhaltungen:
- EP-A2- 1 153 549
- WO-A-01/95741
- WO-A-99/11542
- WO-A1-2007/009568
- DE-A1- 10 042 625
- DE-A1- 10 219 684
- US-B1- 6 200 609
- GARDINER G E ET AL: "Comparative survival rates of human-derived probiotic Lactobacillus paracasei and L. salivarius strains during heat treatment and spray drying." APPLIED AND ENVIRONMENTAL MICROBIOLOGY, Bd. 66, Nr. 6, 2000, Seiten 2605-2612, XP002435984
- N.N.: "Discussionsforum und Portal für ganzheitliche Informationen zur Kaskadenfermentation" , 2007, Retrieved from the Internet: URL:www.archive.org/web/20070125120225/www .kaskadenfermentation.de/Fragen.html>
- N.N.: "Das Buch über Rechtsregulat /"Damit müssen Sie jetzt leben!" "Nein danke")" , 2006, Retrieved from the Internet: URL:www.archive.org/web/20060822013933/rec htsregulate.de/Infos/Inhalt_Buch.html>
- N.N.: "Rechtsregulat" , 2004, Retrieved from the Internet: URL:www.archive.org//web/20040806004953/ww w.niedermaier-pharma.de/siteprodukte/fprod rechtsregulat.htm>
- N.N.: "Rechtsregulat" , 2004, Retrieved from the Internet: URL:www.archive.org//web/20060414144954/ww w.niedermaier-pharma.de/siteprodukte/fprod rechtsregulat.htm>
- N.N.: "Rechts-Regulat" , 2006, Retrieved from the Internet: URL:www.pilz.medica.compaid.at/index.php?o ption=content&task=view&id=69>
- N.N.: "Rechtsregulat (für Borelliose)" , Retrieved from the Internet: URL:www.symptome.ch/vbboard/borreliose/271 8-rechtsregulat-f-r-borelliose.html>

## Beschreibung

Die vorliegende Erfindung betrifft ein flüssiges, fermentiertes und pasteurisiertes Naturprodukt, welches lebende probiotische Mikroorganismen enthält, ein Verfahren zur Herstellung dieses Naturprodukts und einen Behälter für dessen Aufbewahrung.

Als Naturprodukte werden Produkte bezeichnet, die Produkte aus der Natur darstellen, wie beispielsweise die Milch von Tieren. Naturprodukte sind auch solche Produkte, die aus natürlich vorkommenden Rohmaterialien, wie beispielsweise aus Früchten und Gemüse, hergestellt werden.

Seit ein paar Jahren geht der Trend vermehrt zu einer gesunden, ausgewogenen Ernährung. Dabei spielt der Konsum von natürlichen Produkten eine immer größer werdende Rolle.

Auch die Anreicherung von Naturprodukten mit insbesondere probiotischen Bakterien gewinnt in diesem Zusammenhang immer mehr an Bedeutung. Bei probiotischen Bakterien handelt es sich definitionsgemäß um lebensfähige Mikroorganismen, die in der Lage sind, die Gesundheit des Menschen positiv zu beeinflussen.

So kann beispielsweise das Gleichgewicht der Darmflora des Menschen durch verschiedene Belastungen wie Stress, Antibiotika, Alkohol und ballaststoffarme Kost oder bei Erkrankungen der Verdauungsorgane gestört sein, wodurch die natürliche Darmflora nicht mehr in der Lage ist, sich gegen fremde, schädliche Keime zu wehren. Auch Medikamente können eine Änderung der Zusammensetzung des Darmmilieus bewirken, insbesondere Antibiotika und Antimykotika, die die Darmflora zum Teil zerstören.

Probiotische Bakterien helfen dabei, das Gleichgewicht der Darmflora wiederherzustellen und stabil zu halten, indem sie die Zusammensetzung der Darmflora des Menschen verändern und den Teil des Immunsystem positiv beeinflussen, der mit der Darmwand in Verbindung steht. Durch die Produktion von Stoffwechselprodukten, z.B., Milchsäure und Wasserstoffperoxid, verschlechtern probiotische Bakterien beispielsweise die Lebensbedingungen von unerwünschten Mikroorganismen im Darm. Ihre Anwesenheit im Darm verbessert die Verdauungsfunktion. Im Darm produzieren probiotische Bakterien außerdem für den Menschen wichtige Stoffe, wie Niacin, Folsäure und Pyridoxin.

Zu den bekanntesten probiotischen Bakterien gehören die Lactobazillen und Bifidobakterien, die der Ordnung der Milchsäurebakterien angehören. Damit probiotische Bakterien die gewünschten Effekte erzielen, muss deren Konsum gesundheitlich unbedenklich sein. Außerdem müssen sie in größerer Zahl lebend im Darm ankommen, um sich dort anzusiedeln und dürfen nicht durch die Magensäure beim Passieren des Magens zerstört werden.

Aus dem Stand der Technik sind flüssige Naturprodukte bekannt, die probiotische Bakterien enthalten.

So beschreibt die WO 01/58465 A2 den Zusatz von lebenden Lactobacillus-Bakterien oder Bifidobakterien zu einem pharmazeutisch akzeptablen Träger, z.B. Milch, um Darminfektionen von Säuglingen zu behandeln und zu verhindern.

Die US 5,531,988 offenbart eine Trockenzusammensetzung, die eine effektive Menge von nützlichen Mikroorganismen des menschlichen Darms, z.B. Lactobacillus-Bakterien oder Bifidobakterien, und eine effektive Menge einer aktiven Immunglobulinzusammensetzung umfasst, die zur Förderung der Gesundheit des Magen-Darmtrakts des Menschen beiträgt. Diese Trockenzusammensetzung kann konsumiert werden, indem sie vor dem Verzehr in einer Flüssigkeit, z.B. in Wasser oder in Fruchtsaft, suspendiert wird.

Die WO 99/17788 A1 betrifft eine Zusammensetzung zur Behandlung von Pilzerkrankungen, die sowohl lebende als auch tote Milchsäurebakterien umfasst. Die Zusammensetzung kann in flüssiger Form vorliegen und zusätzlich Proteine, Kohlenhydrate, Fette und Vitamine enthalten.

Aus der DE 102 19 648 A1 ist ein Ernährungsprodukt aus einer Basismischung bekannt, die aus Getreide, Hülsenfrüchten, Schmetterlingsblütlern in Verbindung mit Fuchsschwanzgewächsen und/oder in Verbindung mit Gänsefußgewächsen besteht. Diese Mischung ist fermentiert und kann gegebenenfalls pasteurisiert sein. Es wird vorgeschlagen, nach der Fermentation oder kurz vor der Verwendung des Produkts probiotische Stämme hinzuzufügen, aber nur wenn in der vorangegangenen Fermentation Starterkulturen verwendet worden sind, die nicht probiotisch sind. Die Angabe der probiotischen Bakterienstämme ist sehr allgemein gehalten, es gibt auch keine Angabe über die Menge der hinzuzufügenden probiotischen Mikroorganismen.

WO 2007/009568 A1 beschreibt essbare unfermentierte Produkte, die nicht lebende Bakterien enthalten. Die Bakterien werden in mindestens zwei Behandlungen abgetötet. Als Beispiele für diese Produkte sind ein Kräutertee sowie ein Soja-Dessert beschrieben.

Wie eingangs erwähnt, sind Naturprodukte auch solche Produkte, die aus natürlich vorkommenden Rohmaterialien hergestellt werden. Es ist bekannt, dass solche Naturprodukte besonders gut der Steigerung des körperlichen Wohlbefindens und der persönlichen Leistungsfähigkeit des Menschen dienen können, wenn sie mit Hilfe von Mikroorganismen aufgeschlossen werden. Der Vorgang, bei dem natürlich vorkommende Rohmaterialien, z.B. Obst, Gemüse und/oder Hülsenfrüchte, durch Mikroorganismen, z.B. Milchsäurebakterien, abgebaut werden, wird als Fermentation bezeichnet. Durch die Fermentation erhöht sich die Bioverfügbarkeit der verwendeten Rohmaterialien. Dadurch, dass die Rohmaterialien durch das Fermentieren in ihre einzelnen Bestandteile überführt werden, können sie vom Körper besser aufgenommen und verwertet werden, was sich positiv auf den Stoffwechsel des Menschen auswirkt.

Natürliche Fermentgetränke aus Tee, Brot, Milch, Gemüse oder Früchten, die Milchsäurebakterien enthalten, sind ebenfalls bekannt. Die JP 63 251070 A offenbart zum Beispiel ein durch Milchsäurebakterien, z.B. *Lactobacillus bulgaricus, Streptococcus thermophilus* oder *Lactobacillus casei* fermentiertes Fruchtsaftgetränk.

Weiterhin beschreibt die EP 1 153 549 B1 ein flüssiges, durch Mikroorganismen, z.B. *Lactobacillus rhamnosus,* fermentiertes Naturprodukt, welches keine lebenden Bakterien mehr enthält. Es wird durch ein mehrstufiges Fermentationsverfahren hergestellt und besteht aus einer Vielzahl von Abbauprodukten, die für den Menschen verwertbar sind.

Die EP 1 289 380 B1 betrifft ein Verfahren zur Herstellung eines Nahrungsmittelprodukts, z.B. eines Getränks, welches lebensunfähige Lactobacillus-Bakterien beinhaltet, wobei das Verfahren das Zusetzen einer Mischung aus lebensfähigen und lebensunfähigen Lactobacillus-Bakterien, gefolgt vom Inaktivieren der lebensfähigen Bakterien umfasst.

Es hat sich allerdings herausgestellt, dass die bisher bekannten flüssigen, durch Mikroorganismen fermentierten Naturprodukte den Nachteil haben, dass sie trotz eines relativ großen Wirkungsspektrums nicht wirksam genug sind, gerade im Hinblick auf immunstimmulierende Mechanismen im Körper.

Es kann daher als Aufgabe der vorliegenden Erfindung angesehen werden, ein flüssiges, fermentiertes Naturprodukt zur Verfügung zu stellen, welches sich durch ein besonders breites Wirkungsspektrum mit spezieller Aktivierung des Immunsystems auszeichnet.

Es kann weiterhin als Aufgabe angesehen werden, ein Verfahren zur Verfügung zu stellen, mit dem ein flüssiges, fermentiertes Naturprodukt besonders schonend hergestellt werden kann.

Schließlich kann als Aufgabe angesehen werden, einen Behälter zur Aufbewahrung eines flüssigen, fermentierten Naturprodukts zur Verfügung zu stellen, worin das Naturprodukt seine besonders hohe Wirksamkeit nicht verliert.

Diese Aufgaben werden mit den Merkmalen der Patentansprüche 1, 14 und 25 gelöst.

Die vorliegende Erfindung betrifft ein flüssiges, fermentiertes und pasteurisiertes Naturprodukt auf der Basis von Früchten, Gewüse, Hülsenfrüchten, Kräuten und/oder Müssen, welches lebende probiotische Mikroorganismen in einer Menge von 10⁵ bis 10¹⁵ Zellen/ml enthält, wobei der Anteil toter Mikroorganismen im Überschuss vorliegt.

Die Unteransprüche betreffen bevorzugte Ausführungsformen des erfindungsgemäßen Naturprodukts.

Die Erfindung betrifft weiterhin ein Verfahren zur Herstellung eines flüssigen, fermentierten Naturprodukts, welches lebende probiotische Mikroorganismen enthält, wobei der Anteil toter Mikroorganismen im Überschuss vorliegt, mit den Stufen: (i) Zur Verfügung stellen eines flüssigen, fermentierten und pasteurisierten Naturprodukts auf der Basis von Früchten, Gewüse, Hülsenfrüchten, Kräuten und/oder Müssen, (ii) Hinzufügen von probiotischen Mikroorganismen in einer Menge von 10⁵ bis 10¹⁵ Zellen/ml zu dem flüssigen, fermentierten und pasteurisierten Naturprodukt und (iii) Vermischen der probiotischen Mikroorganismen mit dem flüssigen, fermentierten und pasteurisierten Naturprodukt unmittelbar vor dem Verzehr.

Die Unteransprüche betreffen bevorzugte Ausführungsformen des erfindungsgemäßen Verfahrens.

Außerdem betrifft die Erfindung einen Behälter für die getrennte Aufbewahrung des Obigen Naturprodukts und den den lebenden probiotischen Mikroorganismen, wobei der Behälter wenigstens zwei Kammern aufweist und in der ersten Kammer das flüssige, fermentierte und pasteurisierte Naturprodukt und in der zweiten Kammer mindestens die probiotischen Mikroorganismen enthalten sind.

Die Unteransprüche betreffen bevorzugte Ausführungsformen des erfindungsgemäßen Behälters.

Es hat sich überraschenderweise herausgestellt, dass mit dem erfindungsgemäßen flüssigen, fermentierten und pasteurisierten Naturprodukt die positiven Wirkungen von lebenden probiotischen Mikroorganismen und von toten Mikroorganismen auf den menschlichen Organismus miteinander kombiniert und somit durch einen einzigartigen Synergismus potenziert werden können.

Die positiven Wirkungen des erfindungsgemäßen Naturprodukts lassen sich im Einzelnen wie folgt beschreiben:
So können beispielsweise die vielfältigen Abbauprodukte in einem fermentierten Naturprodukt vom menschlichen Körper viel leichter verwertet werden, was positive Auswirkungen auf den Stoffwechsel des Menschen hat. Die verbesserte Bioverfügbarkeit eines fermentierten Naturprodukts steigert außerdem das allgemeine Wohlbefinden und die körperliche Leistungsfähigkeit des Menschen.

Die Verabreichung von lebenden probiotischen Mikroorganismen fördert die Gesundheit des Magen-DarmTrakts. Beispielsweise können sich probiotische Bakterien im Darm ansiedeln und auf diese Weise den Teil des Immunsystem stärken, welcher mit der Darmwand in Verbindung steht. Durch die Bildung von Stoffwechselprodukten, wie zum Beispiel Milchsäure und Wasserstoffperoxid, sind probiotische Bakterien in der Lage, die Lebensbedingungen von unerwünschten, gesundheitsschädlichen Mikroorganismen zu verschlechtern und somit das mikrobiologische Gleichgewicht im Darm in Richtung gesundheitsfördernde Mikroorganismen zu verschieben. Probiotische Bakterien verkürzen oder vermindern Durchfallerkrankungen und fördern die Milchzuckerverdauung. Darüber hinaus sind sie hilfreich bei chronischen entzündlichen Darmerkrankungen. Studien haben gezeigt, dass probiotische Bakterien auch wirksam gegen Allergien sind. Außerdem können sie den Cholesterinspiegel senken, das Risiko von Herzkranzgefäßerkrankungen reduzieren und die Zahl von Pilzinfektionen (Mykosen) vermindern.

Auch tote Mikroorganismen führen zu einer Steigerung der Immunkraft des menschlichen Organismus. So sind insbesondere die Zellwand- und Zellbestandteile von toten Bakterien, wie beispielsweise Peptide, Glucane, Phospholipide, Polysaccharide und die Substanz Murein in der Lage, das Immunsystem des Menschen zu stimulieren. Dieser immunstimmulierende Effekt erklärt sich dadurch, dass die abgetöteten Bakterien, den angeborenen, jederzeit zur Verfügung stehenden Teil der körpereigenen Abwehr aktivieren. Sie wirken im Köper als eine Art Alarmsignal für Eindringlinge, woraufhin das Immunsystem mobilisiert wird. Im Zuge dieser Mobilisierung wird beispielsweise die Aktivität von Makrophagen gesteigert. Außerdem werden die Leukozyten in Habachtstellung gebracht. Eine überschießende Reaktion des Immunsystems wird jedoch durch das Eintreten einer entzündungshemmenden Wirkung gleich einem metabolischen Pendel verhindert. Außerdem wird angenommen, dass den Bestandteilen der toten Bakterien eine prebiotische Rolle zukommt. Prebiotische Stoffe sind in der Lage, das Wachstum ganz bestimmter Bakterien des menschlichen Darms zu fördern. Vereinfacht wird angenommen, dass die Bestandteile der toten Bakterien den probiotischen Bakterien im Darm als Nahrung dienen.

Das erfindungsgemäße Naturprodukt, welches die Wirkung eines fermentierten Produkts und die Wirkung von toten und lebenden probiotischen Mikroorganismen synergistisch vereint, ist somit besonders wohltuend und wirksam für den menschlichen Organismus. Es zeichnet sich durch ein besonders breites Wirkungsspektrum aus.

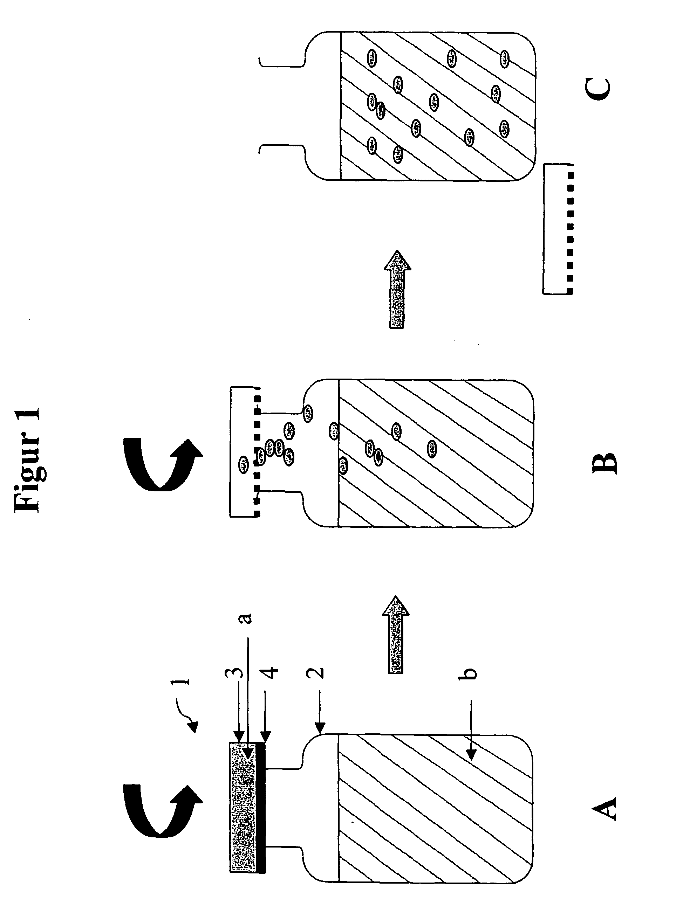

Figur 1 zeigt ein Beispiel für einen Behälter zur Aufbewahrung des erfindungsgemäßen Naturprodukts.

Es hat sich als besonders vorteilhaft für die Wirkung auf den menschlichen Organismus erwiesen, wenn das erfindungsgemäße Naturprodukt lebende probiotische Mikroorganismen in einer Menge von 10⁵ bis 10¹⁵ Zellen/ml enthält und der Anteil toter Mikroorganismen (im Verhältnis zu den lebenden Bakterien) im Überschuss vorliegt.

Das erfindungsgemäße Naturprodukt enthält vorzugweise die probiotischen Mikroorganismen in einer Menge von 10⁶ bis 10¹² Zellen/ml. Besonders bevorzugt ist eine Menge von 10⁸ bis 10¹⁰ Zellen/ml.

Das erfindungsgemäße Naturprodukt ist pasteurisiert. Ein solches Produkt kann beispielsweise nach dem in der EP 1 153 549 B1 beschriebenen Verfahren hergestellt werden.

Das erfindungsgemäße Naturprodukt besteht vorzugsweise aus einem Ferment oder aus einem Fermentauszug von Rohmaterialien. Dabei ist unter einem Ferment von Rohmaterialien ein Stoffgemisch zu verstehen, welches sowohl lösliche als auch unlösliche Bestandteile enthält. Es hat den Vorteil, dass es reich an wasserunlöslichen Ballaststoffen ist. Das ungereinigte Ferment stellt ein Konzentrat dar, das für den Konsumenten besonders gehaltvoll ist und sich als gesunde Energiequelle eignet. Ein Fermentauszug von Rohmaterialien stellt wiederum ein durch beispielsweise Zentrifugieren und/oder Filtrieren gereinigtes Filtrat dar. Aus einem solchen Filtrat sind die unlöslichen Bestandteile entfernt. Es stellt eine naturtrübe Flüssigkeit dar.

Das flüssige, fermentierte Naturprodukt basiert auf Früchten, Gemüse, Hülsenfrüchten, Kräutern und/oder Nüssen jeder Art. Die Mischung an sich wird als nicht kritisch angesehen. Es ist praktisch jede Variation aus Früchten, Gemüse und Hülsenfrüchten möglich. Bevorzugt werden Rohmaterialien aus ökologisch kontrolliertem Anbau verwendet.

Vorzugsweise sind die probiotischen Mikroorganismen im erfindungsgemäßen Naturprodukt in beispielsweise lyophilisierter Form enthalten.

In einer bevorzugten Ausführungsform umfasst das erfindungsgemäße Naturprodukt probiotische Mikroorganismen, die aus Bakterien, Pilzen, Hefen und/oder Mischungen daraus ausgewählt sind. Besonders bevorzugt sind Bakterien enthalten.

Das erfindungsgemäße Naturprodukt umfasst bevorzugt als probiotische Bakterien mindestens ein Bakterium der Ordnung *Lactobacillales* (Milchsäurebakterien). In einer alternativen Ausführungsform der Erfindung liegen die Mikroorganismen in dem Naturprodukt als Gemisch aus *Lactobacillales* mit anderen probiotischen Mikroorganismen vor.

*Lactobacillales* sind grampositive, stets anaerobe aber meist aerotolerante Bakterien, die sich dadurch auszeichnen, dass sie Zucker zu Milchsäure abbauen. Sie gehören zu den wichtigsten Vertretern der menschlichen Darmflora. Daher kann besonders die Verabreichung von *Lactobacillales* das Gleichgewicht der Darmflora wiederherstellen und stabil halten. Das erfindungsgemäße Naturprodukt, welches *Lactobacillales* umfasst, kann somit der Steigerung des körperlichen Wohlbefindens des Menschen dienen.

Der Ordnung *Lactobacillales* gehören die Familien *Lactobacillaceae, Aerococcaceae, Carnobacteriaceae, Enterococcaceae, Leuconostocaceae* und *Streptococcaceae* an.

Vorzugsweise enthält das erfindungsgemäße Naturprodukt Bakterien der Familie *Lactobacillaceae.* Besonders bevorzugt umfasst das erfindungsgemäße Naturprodukt Bakterien der Gattung *Lactobacillus.* Noch mehr bevorzugt sind in dem erfindungsgemäßen Naturprodukt Bakterien der Art *Lactobacillus rhamnosus* enthalten. Überraschender Weise hat sich die Verabreichung von Bakterien der Art *Lactobacillus rhamnosus* in einer Vollblutstudie als besonders immunsteigernd erwiesen. Das erfindungsgemäße Naturprodukt, welches diese Bakterienart enthält, ist demnach hoch wirksam.

Am meisten bevorzugt umfasst das erfindungsgemäße Naturprodukt Bakterien der Art *Lactobacillus rhamnosus* und mindestens einen anderen probiotischen Mikroorganismus in Abhängigkeit vom jeweiligen Wirkungsspektrum.

Vorzugsweise enthält das erfindungsgemäße Naturprodukt weiterhin Additive, wie beispielsweise sekundäre Pflanzenstoffe, z.B. Lutein, Vitamine, Mineralien, Fettsäuren, Polysaccharide, Polyglucane und/oder Extrakte aus Pilzen.

Gemäß der Erfindung weist das Verfahren zur Herstellung eines flüssigen, fermentierten und pasteurisierten Naturprodukts, welches lebende probiotische Mikroorganismen enthält, wobei der Anteil toter Mikroorganismen im Überschuss vorliegt, insbesondere eines erfindungsgemäßen Naturprodukts, die Stufen auf: (i) zur Verfügung stellen eines flüssigen, fermentierten und pasteurisierten Naturprodukts, (ii) Hinzufügen von probiotischen Mikroorganismen in einer Menge von 10⁵ bis 10¹⁵ Zellen/ml zu dem flüssigen, fermentierten Naturprodukt und (iii) Vermischen der probiotischen Mikroorganismen mit dem flüssigen, fermentierten und pasteurisierten Naturprodukt unmittelbar vor dem Verzehr.

Das erfindungsgemäße Verfahren wird vorzugsweise mit probiotischen Mikroorganismen in einer Menge von 10⁶ bis 10¹² Zellen/ml durchgeführt, weil sich diese Menge als besonders wirksam für den menschlichen Organismus herausgestellt hat. Besonders bevorzugt wird das erfindungsgemäße Verfahren mit probiotischen Mikroorganismen in einer Menge von 10⁸ bis 10¹⁰ Zellen/ml durchgeführt, weil sich diese Menge als hochwirksam für den menschlichen Organismus herausgestellt hat.

Unter unmittelbar vor dem Verzehr ist ein Zeitraum von einigen Sekunden bis zu 16 Stunden zu verstehen, die zwischen dem Herstellen des Naturprodukts und dessen Konsum liegen können. Üblicherweise wird das Naturprodukt innerhalb von einer Minute bis maximal 10 Minuten konsumiert, z.B. in Form einer Tagesdosis. Das Naturprodukt kann aber auch über den Tag verteilt, beispielsweise als Teildosis am Morgen und Abend, in einem. Abstand von bis zu 16 Stunden, eingenommen werden. Da erst kurz vor dem Verzehr die lebenden probiotischen Mikroorganismen zu dem flüssigen, fermentierten Naturprodukt gegeben werden, ist eine hohe Wirksamkeit garantiert.

Mit Hilfe des erfindungsgemäßen Verfahrens lässt sich ein flüssiges, fermentiertes und pasteurisiertes Naturprodukt, welches probiotische Mikroorganismen enthält, besonders schonend herstellen.

Durch das erfindungsgemäße Verfahren wird ein flüssiges, fermentiertes und pasteurisiertes Naturprodukt hergestellt, welches sowohl lebende als auch tote Mikroorganismen enthält. Der Anteil toter Mikroorganismen liegt dabei im Überschuss vor. Ein solches Naturprodukt ist besonders wirksam. Wie bereits beschrieben, wirkt sich gerade die Kombination von lebenden und toten Bakterien besonders stimulierend auf das Immunsystem des Menschen aus. Während lebende Bakterien probiotisch auf den menschlichen Organismus wirken, wird angenommen, dass den Bestandteilen der toten Bakterien eine prebiotische Wirkung zugeschrieben werden kann. Darüber hinaus können die vielfältigen Bestandteile eines fermentierten Naturprodukts zur Steigerung der Stoffwechselaktivität des Menschen beitragen.

Das in dem erfindungsgemäßen Verfahren verwendete Naturprodukt ist pasteurisiert.

Unter Pasteurisierung wird allgemein das kurzzeitige Erhitzen von Substanzen auf 60 bis 90°C zur Abtötung von Mikroorganismen verstanden. Ein solches Produkt kann beispielsweise nach dem in der EP 1 153 549 B1 beschriebenen Verfahren hergestellt werden. In der EP 1 153 549 B1 kann das Pasteurisieren am Ende des Fermentationsprozesses durchgeführt werden, um alle lebenden Mikroorganismen im fermentierten Produkt abzutöten.

Vorteilhaft ist, dass durch das Pasteurisieren die Haltbarkeit des Naturprodukts erhöht wird. Das Naturprodukt verändert beim Pasteurisieren seine Eigenschaften nicht, Konsistenz und Geschmack bleiben erhalten.

In dem erfindungsgemäßen Verfahren werden bevorzugt Mikroorganismen in lyophilisierter Form hinzugefügt, weil lyophilisierte Mikroorganismen eine gute Überlebensrate während der Lagerung zeigen. Um die Vitalität der Mikroorganismen so lange wie möglich hoch zu halten, werden die Mikroorganismen daher erst unmittelbar vor dem Verzehr einem flüssigen fermentierte Naturprodukt hinzugefügt.

In einer bevorzugten Ausführungsform des erfindungsgemäßen Verfahrens werden die Mikroorganismen aus Bakterien, Pilzen, Hefen und/oder Mischungen daraus gewählt. Besonders bevorzugt werden Bakterien verwendet.

In dem erfindungsgemäßen Verfahren werden bevorzugt als probiotische Bakterien mindestens ein Bakterium der Ordnung *Lactobacillales* (Milchsäurebakterien) einem flüssigen, fermentierten Naturprodukt hinzugefügt. Milchsäurebakterien gehören zu den wichtigsten Vertretern der menschlichen Darmflora. Es hat sich gezeigt, dass die Substitution von mindestens einem Bakterium der Ordnung Bakterien *Lactobacillales* das Gleichgewicht einer gestörten Darmflora wiederherstellen und stabil halten kann. Besonders gesundheitsfördernd ist es, mindestens ein Bakterium der Ordnung *Lactobacillales* zur Vorbeugung von Magen-Darm Krankheiten und zur Stimulierung des Immunsystems einzunehmen.

Vorzugsweise werden Bakterien der Familie *Lactobacillacea* hinzugegeben. Besonders bevorzugt werden in dem erfindungsgemäßen Verfahren Bakterien der Gattung *Lactobacillus* verwendet. Am meisten bevorzugt werden Bakterien der Art *Lactobacillus rhamnosus* in dem erfindungsgemäßen Verfahren eingesetzt, weil sie sich als besonders immunfördernd herausgestellt haben.

In einer besonderen Ausführungsform des erfindungsgemäßen Verfahrens wird mindestens ein probiotischer Mikroorganismus der Ordnung *Lactobacillales* im Gemisch mit mindestens einem anderen probiotischen Mikroorganismus eingesetzt. Als hoch wirksam hat sich die Kombination von Bakterien der Art *Lactobacillus rhamnosus* mit mindestens einem anderen probiotischen Mikroorganismus in Abhängigkeit vom jeweiligen Wirkungsspektrum herausgestellt.

Die Erfindung betrifft weiterhin einen Behälter für die getrennte Aufbewahrung des oben beschriebenen Naturprodukts und der lebenden probiotischen Mikroorganismen, wobei der Behälter wenigstens zwei Kammern aufweist und in der ersten Kammer das flüssige, fermentierte Naturprodukt und in der zweiten Kammer mindestens die probiotischen Mikroorganismen enthalten sind.

Vorzugsweise enthält die zweite Kammer weiterhin Additive, wie beispielsweise sekundäre Pflanzenstoffe, z.B. Lutein, Vitamine, Mineralien Fettsäuren, Polysaccharide, Polyglucane und/oder Extrakte aus Pilzen. Diese Bestandteile liegen bevorzugt in lyophilisierter Form vor. Es hat sich gezeigt, dass der Zusatz von sekundären Pflanzenstoffen, z.B. Lutein, Vitaminen, Mineralien, Fettsäuren, Polysacchariden, Polyglucanen und/oder Extrakten aus Pilzen, die Wirksamkeit des erfindungsgemäßen Naturprodukts weiter steigert. Die Zusammenstellung dieser Bestandteile erfolgt individuell und richtet sich nach dem gewünschten Wirkungsspektrum. Beispielsweise spielt bei der Auswahl der einzelnen Bestandteile der Konsumentenkreis eine Rolle. Während Frauen einen erhöhten Bedarf an Folsäure, Eisen, Magnesium, Zink und Biotin haben, bietet sich die Substitution von Vitamin K, B5, B9, B2 bei Männern an. Sportler haben einen erhöhten Bedarf an Natrium, Calcium, Magnesium, Zink, Vitamin E und C.

Eine der beiden Kammern ist vorzugsweise topfförmig ausgebildet und die andere der beiden Kammern wird durch einen Verschluss gebildet, mit dem die erste Kammer abschließbar ist. Beispielsweise kann der Verschluss innen ein Schraubgewinde zum Verschrauben aufweisen.

Die Kammer, die durch den Verschluss gebildet wird, ist vorzugsweise mit einer Trennschicht verschlossen. Die Trennschicht kann aus einem pflanzlichen faserhaltigen Material oder aus Kunststoff gebildet sein. Vorzugsweise besteht die Trennschicht aus Papier oder aus einer Folie aus Kunststoff, da diese Materialien eine geringe Reißfestigkeit aufweisen.

Der Behälter kann aus Glas oder Kunststoff bestehen. Besonders bevorzugt ist Glas, weil das flüssige, fermentierte Naturprodukt farbliche Rückstände an Kunststoff hinterlassen kann. Darüber hinaus kann der Behälter aus jedem anderen Material gebildet sein, vorausgesetzt, dass es sich zur Aufbewahrung des erfindungsgemäßen Naturprodukts eignet.

Der Behälter kann in jeder beliebigen Form vorliegen. Vorzugsweise werden Flaschen verwendet, um das erfindungsgemäße Naturprodukt aufzubewahren, da sie sich besonders gut handhaben lassen.

Der erfindungsgemäße Behälter hat den Vorteil, dass probiotische Bakterien und ein flüssiges, fermentiertes und pasteurisiertes Naturprodukt getrennt voneinander aufgewahrt werden können. Erst kurz vor dem Verzehr, beim Öffnen des Behälters, werden probiotische Bakterien zu einem flüssigen, fermentierten Naturprodukt hinzugefügt, wodurch das erfindungsgemäße Naturprodukt hergestellt wird. Folglich sind die einzelnen Bestandteile des Naturprodukts in dem erfindungsgemäßen Behälter besonders lang haltbar ohne dass deren Wirksamkeit abnimmt.

Wie bereits beschrieben, steigert ein fermentiertes, flüssiges und pasteurisiertes Naturprodukts, welches sich in der ersten Kammer des erfindungsgemäßen Behälters befindet, durch seine hohe Bioverfügbarkeit das allgemeine Wohlbefinden und die körperliche Leistungsfähigkeit des Menschen. Die darin enthaltenen toten Mikroorganismen steigern zudem die Immunkraft des Menschen. Außerdem wird angenommen, dass den Bestandteilen toter Bakterien eine prebiotische Wirkung zuzuschreiben ist. Die probiotischen Mikroorganismen, die die zweite Kammer des erfindungsgemäßen Behälters umfasst, sind sowohl immunsteigernd als auch für die Gesundheit des Magen-Darm Trakts förderlich. Durch das Öffnen des Behälters vereinigen sich die probiotischen Mikroorganismen aus der zweiten Kammer mit dem flüssigen, fermentierten und pasteurisierten Naturprodukt in der ersten Kammer und damit auch deren positive Wirkungen. Das durch diesen Vorgang hergestellte erfindungsgemäße Naturprodukt zeichnet sich daher durch seine besonders hohe Wirksamkeit aus.

In Figur 1 ist ein Ausführungsbeispiel zur Aufbewahrung des erfindungsgemäßen Naturprodukts dargestellt. Figur 1A zeigt als erfindungsgemäßen Behälter 1 eine verschließbare Flasche mit einem Verschluss. Die Flasche weist zwei Kammern 2,3 auf. Die erste Kammer 2 ist topfförmig. Sie wird im Ausführungsbeispiel durch die Flasche gebildet. Die Flasche hat ein Schraubgewinde. Sie enthält das flüssige, fermentierte und pasteurisierte Naturprodukt b. Die zweite Kammer 3 wird durch einen Verschluss gebildet. Der Verschluss ist im Ausführungsbeispiel ein Verschluss zum Schrauben. Die zweite Kammer 3 ist durch eine Trennschicht 4 verschlossen. Im Ausführungsbeispiel besteht die Trennschicht aus Papier. In dem Hohlraum des Verschlusses sind die probiotischen Bakterien a enthalten.

Der Behälter 1 ist so ausgestaltet, dass durch eine Relativbewegung zwischen den beiden Kammern 2 und 3, hier also zwischen der Flasche und dem Verschluss, z.B. durch Drehen, die zweite Kammer 3, beispielsweise durch Zerstören der Trennschicht, geöffnet werden kann (siehe Figur 1B). Auf diese Weise können die probiotischen Bakterien, die im Verschluss enthalten sind, in das flüssige, fermentierte und pasteurisierte Naturprodukt gegeben werden. Der Behälter 1 ist weiterhin so ausgestaltet, dass durch das Fortsetzen der Relativbewegung, z.B. durch weiteres Drehen, die zweite Kammer 3, im Ausführungsbeispiel der Verschluss, von der ersten Kammer 2, im Ausführungsbeispiel die Flasche, vollständig getrennt werden kann (siehe Figur 1C). Aus dem so geöffneten Behälter 1 kann das flüssige, fermentierte und pasteurisierte Naturprodukt, welches probiotische Bakterien enthält, konsumiert werden. Der Behälter kann auf entsprechende Weise wieder verschlossen werden.

Der Behälter 1 kann ein Fassungsvermögen von 10 bis 350 ml haben. Vorzugsweise kann der Behälter 1 ein Fassungsvermögen von 10 bis 30 ml besitzen. Besonders bevorzugt ist ein Behälter 1 mit einem Fassungsvermögen von 20 ml, weil diese Menge der empfohlenen Tagesdosis des erfindungsgemäßen Naturprodukts entspricht. Ein solcher Behälter 1 hat den Vorteil, dass nach dem Öffnen der gesamte Inhalt verbraucht wird. Das erfindungsgemäße Naturprodukt kann somit durch weitere Lagerung nicht an Wirksamkeit einbüßen.

Das erfindungsgemäße Naturprodukt ist ein biologisches Produkt. Es kann sowohl innerlich als auch äußerlich verabreicht werden. Für die innere Verabreichung wird es in der Regel oral als Lebensmittel, Nahrungsmittel, Nahrungsergänzungsmittel, diätetisches Lebensmittel oder als bilanzierte Diät gegeben. Von dem erfindungsgemäßen Naturprodukt werden in der Regel 20 ml pro Tag (Tagesdosis) eingenommen. Diese Dosis kann auch geteilt werden und beispielsweise als Teildosis am Morgen und Abend konsumiert werden.

Es hat sich herausgestellt, dass das erfindungsgemäße Naturprodukt auch äußerlich auf der Haut angewendet werden kann. Hier hat es sich als besonders günstig erwiesen, wenn das flüssige, fermentierte und pasteurisierte Naturprodukt in Wickel, Kompressen eingegeben wird und dann auf die betroffenen Hautstellen gelegt wird. Es kann auch als Sprühlösung direkt aufgetragen werden.

Die Erfindung wird anhand des nachfolgenden Beispiels, allerdings ohne Einschränkung darauf, veranschaulicht.

### Beispiel

Das flüssige, fermentierte und pasteurisierte Naturprodukt wird nach dem in der EP 1 153 549 B1 beschriebenen Verfahren hergestellt. Zu diesem flüssigen, fermentierten und pasteurisierten Naturprodukt werden unmittelbar vor dem Verzehr lebende Bakterien der Art *Lactobacillus rhamnosus* oder ein Bakteriengemisch mit *Lactobacillus rhamnosus* in einer Menge von 10⁹ Zellen/ml gegeben. Die probiotischen Bakterien oder das Bakteriengemisch werden anschließend mit dem flüssigen, fermentierten Naturprodukt vorsichtig vermischt. Das Produkt liegt somit zum sofortigen Verzehr vor.

## Patentansprüche

1. Flüssiges, fermentiertes und pasteurisiertes Naturprodukt auf der Basis von Früchten, Gemüse, Hülsenfrüchten, Kräutern und/oder Nüssen, welches lebende probiotische Mikroorganismen in einer Menge von 10⁵ bis 10¹⁵ Zellen/ml enthält, wobei der Anteil toter Mikroorganismen im Überschuss vorliegt.

2. Naturprodukt nach Anspruch 1, **dadurch gekennzeichnet, dass** es die probiotischen Mikroorganismen in einer Menge von 10⁶ bis 10¹² Zellen/ml enthält.

3. Naturprodukt nach Anspruch 2, **dadurch gekennzeichnet, dass** es die probiotischen Mikroorganismen in einer Menge von 10⁸ bis 10¹⁰ Zellen/ml enthält.

4. Naturprodukt nach mindestens einem der Ansprüche 1 bis 3, **dadurch gekennzeichnet, dass** es aus einem Ferment oder aus einem Fermentauszug von Rohmaterialien besteht.

5. Naturprodukt nach mindestens einem der Ansprüche 1 bis 4, **dadurch gekennzeichnet, dass** die probiotischen Mikroorganismen aus Bakterien, Pilzen, Hefen und/oder Mischungen daraus ausgewählt sind.

6. Naturprodukt nach Anspruch 5, **dadurch gekennzeichnet, dass** die probiotischen Mikroorganismen Bakterien sind.

7. Naturprodukt nach mindestens einem der Ansprüche 1 bis 6, **dadurch gekennzeichnet, dass** es probiotische Mikroorganismen in lyophilisierter Form enthält.

8. Naturprodukt nach mindestens einem der Ansprüche 5 bis 7, **dadurch gekennzeichnet, dass** die probiotischen Bakterien mindestens ein Bakterium der Ordnung *Lactobacillales* umfassen.

9. Naturprodukt nach Anspruch 8, **dadurch gekennzeichnet, dass** die genannten Bakterien Bakterien der Familie *Lactobacillacea* sind.

10. Naturprodukt nach Anspruch 9, **dadurch gekennzeichnet, dass** die genannten Bakterien Bakterien der Gattung *Lactobacillus* sind.

11. Naturprodukt nach Anspruch 10, **dadurch gekennzeichnet, dass** die genannten Bakterien Bakterien der Art *Lactobacillus rhamnosus* sind.

12. Naturprodukt nach mindestens einem der Ansprüche 1 bis 11, **dadurch gekennzeichnet, dass** die Mikroorganismen als Gemisch aus *Lactobacillales* mit anderen probiotischen Mikroorganismen vorliegen.

13. Naturprodukt nach mindestens einem der Ansprüche 1 bis 12, **dadurch gekennzeichnet, dass** es weiterhin sekundäre Pflanzenstoffe, Vitamine, Mineralien, Fettsäuren, Polysaccharide, Polyglucane und/oder Extrakte aus Pilzen enthält.

14. Verfahren zur Herstellung eines flüssigen, fermentierten Naturprodukts nach mindestens einem der vorangegangenen Ansprüche 1 bis 13, mit den Stufen:
(i) Zur Verfügung stellen eines flüssigen, fermentierten und pasteurisierten Naturprodukts auf der Basis von Früchten, Gemüse, Hülsenfrüchte, Kräutern und/oder Nüssen,
(ii) Hinzufügen von probiotischen Mikroorganismen in einer Menge von 10⁵ bis 10¹⁵ Zellen/ml zu dem flüssigen, fermentierten und pasteurisierten Naturprodukt und
(iii) Vermischen der probiotischen Mikroorganismen mit dem flüssigen, fermentierten und pasteurisierten Naturprodukt
unmittelbar vor dem Verzehr.

15. Verfahren nach Anspruch 14, **dadurch gekennzeichnet, dass** die probiotischen Mikroorganismen in einer Menge von 10⁶ bis 10¹² Zellen/ml dem flüssigen, fermentierten Naturprodukt hinzugefügt werden.

16. Verfahren nach Anspruch 15, **dadurch gekennzeichnet, dass** die probiotischen Mikroorganismen in einer Menge von 10⁸ bis 10¹⁰ Zellen/ml dem flüssigen, fermentierten Naturprodukt hinzugefügt werden.

17. Verfahren nach mindestens einem der Ansprüche 14 bis 16, **dadurch gekennzeichnet, dass** als probiotische Mikroorganismen Bakterien, Pilze, Hefen und/oder Mischungen daraus hinzugefügt werden.

18. Verfahren nach Anspruch 17, **dadurch gekennzeichnet, dass** als probiotische Mikroorganismen Bakterien hinzugefügt werden.

19. Verfahren nach mindestens einem der Ansprüche 14 bis 18, **dadurch gekennzeichnet, dass** die probiotischen Mikroorganismen in lyophilisierter Form hinzugefügt werden.

20. Verfahren nach mindestens einem der Ansprüche 17 bis 19, **dadurch gekennzeichnet, dass** die hinzugefügten probiotischen Bakterien mindestens ein Bakterium der Ordnung *Lactobacillales* umfassen.

21. Verfahren nach Anspruch 20, **dadurch gekennzeichnet, dass** die hinzugefügten Bakterien Bakterien der Familie *Lactobacillacea* sind.

22. Verfahren nach Anspruch 21, **dadurch gekennzeichnet, dass** die hinzugefügten Bakterien Bakterien der Gattung *Lactobacillus* sind.

23. Verfahren nach Anspruch 22, **dadurch gekennzeichnet, dass** die hinzugefügten Bakterien Bakterien der Art *Lactobacillus rhamnosus* sind.

24. Verfahren nach mindestens einem der Ansprüche 14 bis 23, **dadurch gekennzeichnet, dass** mindestens ein Mikroorganismus der Ordnung *Lactobacillales* im Gemisch mit mindestens einem anderen probiotischen Mikroorganismus eingesetzt wird.

25. Behälter (1) für die getrennte Aufbewahrung eines flüssigen, fermentierten und pasteurisierten Naturprodukts und lebenden probiotischen Mikroorganismen zur Herstellung eines Naturprodukts nach mindestens einem der vorangegangenen Ansprüche 1 bis 13, wobei der Behälter (1) wenigstens zwei Kammern (2, 3) aufweist und in der ersten Kammer (2) das flüssige, fermentierte und pasteurisierte Naturprodukt und in der zweiten Kammer (3) mindestens die probiotischen Mikroorganismen in einer Menge enthalten sind, dass das Naturprodukt nach Zugabe der lebenden probiotische Mikroorganismen diese in einer Menge von 10⁵ bis 10¹⁵ Zellen/ml enthält und wobei durch eine Relativbewegung zwischen den Kammern (2 ,3) die zweite Kammer (3) zu öffnen ist.

26. Behälter nach Anspruch 25, **dadurch gekennzeichnet, dass** eine der beiden Kammern (2, 3) topfförmig ist und die andere der beiden Kammern durch einen Verschluss gebildet ist, mit dem die erste Kammer (2) abschließbar ist.

27. Behälter nach Anspruch 25 und/oder 26, **dadurch gekennzeichnet, dass** die zweite Kammer (3) eine Trennschicht (4) aufweist.

28. Behälter nach mindestens einem der Ansprüche 25 bis 27, **dadurch gekennzeichnet, dass** die erste Kammer (2) und die zweite Kammer (3) ein Schraubgewinde aufweisen.

29. Behälter nach mindestens einem der Ansprüche 25 bis 28, **dadurch gekennzeichnet, dass** in der zweiten Kammer (3) weiterhin sekundäre Pflanzenstoffe, Vitamine, Mineralien, Fettsäuren, Polysaccharide, Polyglucane und/oder Extrakte aus Pilzen enthalten sind.

30. Verwendung des Naturprodukts nach mindestens einem der Ansprüche 1 bis 13 zur inneren und äußeren Verabreichung.

31. Verwendung nach Anspruch 30 als Lebens- und Nahrungsmittel.

32. Verwendung nach Anspruch 30 in einem Wickel oder einer Kompresse.

## Claims

1. Liquid fermented and pasteurized natural product based on fruits, vegetables, pulses, herbs and/or nuts that contains living probiotic microorganisms in a quantity from 10⁵ to 10¹⁵ cells/ml, the proportion of dead microorganisms being present in excess.

2. Natural product according to Claim 1, **characterised in that** it contains the probiotic microorganisms in a quantity from 10⁶ to 10¹² cells/ml.

3. Natural product according to Claim 2, **characterised in that** it contains the probiotic microorganisms in a quantity from 10⁸ to 10¹⁰ cells/ml.

4. Natural product according to at least one of Claims 1 to 3, **characterised in that** it consists of a ferment or of a ferment extract of raw materials.

5. Natural product according to at least one of Claims 1 to 4, **characterised in that** the probiotic microorganisms are selected from bacteria, fungi, yeasts and/or mixtures thereof.

6. Natural product according to Claim 5, **characterised in that** the probiotic microorganisms are bacteria.

7. Natural product according to at least one of Claims 1 to 6, **characterised in that** it contains probiotic microorganisms in lyophilised form.

8. Natural product according to at least one of Claims 5 to 7, **characterised in that** the probiotic bacteria include at least one bacterium of the order Lactobacillales.

9. Natural product according to Claim 8, **characterised in that** the named bacteria are bacteria of the family Lactobacillacea.

10. Natural product according to Claim 9, **characterised in that** the named bacteria are bacteria of the genus Lactobacillus.

11. Natural product according to Claim 10, **characterised in that** the named bacteria are bacteria of the species *Lactobacillus rhamnosus.*

12. Natural product according to at least one of Claims 1 to 11, **characterised in that** the microorganisms are present in the form of a mixture of Lactobacillales with other probiotic microorganisms.

13. Natural product according to at least one of Claims 1 to 12, **characterised in that** it further contains secondary plant substances, vitamins, minerals, fatty acids, polysaccharides, polyglucanes and/or extracts from fungi.

14. Process for producing a liquid fermented natural product according to at least one of the preceding Claims 1 to 13, with the following stages:
(i) making available a liquid fermented and pasteurized natural product based on fruits, vegetables, pulses, herbs and/or nuts,
(ii) adding probiotic microorganisms in a quantity from 10⁵ to 10¹⁵ cells/ml to the liquid fermented and pasteurized natural product and
(iii) mixing the probiotic microorganisms with the liquid fermented and pasteurized natural product immediately prior to consumption.

15. Process according to Claim 14, **characterised in that** the probiotic microorganisms are added in a quantity from 10⁶ to 10¹² cells/ml to the liquid fermented natural product.

16. Process according to Claim 15, **characterised in that** the probiotic microorganisms are added in a quantity from 10⁸ to 10¹⁰ cells/ml to the liquid fermented natural product.

17. Process according to at least one of Claims 14 to 16, **characterised in that** bacteria, fungi, yeasts and/or mixtures thereof are added by way of probiotic microorganisms.

18. Process according to Claim 17, **characterised in that** bacteria are added by way of probiotic microorganisms.

19. Process according to at least one of Claims 14 to 18, **characterised in that** the probiotic microorganisms are added in lyophilised form.

20. Process according to at least one of Claims 17 to 19, **characterised in that** the added probiotic bacteria include at least one bacterium of the order Lactobacillales.

21. Process according to Claim 20, **characterised in that** the added bacteria are bacteria of the family Lactobacillacea.

22. Process according to Claim 21, **characterised in that** the added bacteria are bacteria of the genus Lactobacillus.

23. Process according to Claim 22, **characterised in that** the added bacteria are bacteria of the species Lactobacillus rhamnosus.

24. Process according to at least one of Claims 14 to 23, **characterised in that** at least one microorganism of the order Lactobacillales is employed in a mixture with at least one other probiotic microorganism.

25. Container (1) for separately holding a liquid fermented and pasteurized natural product and living probiotic microorganismus for the production of a natural product according to at least one of the preceding Claims 1 to 13, the container (1) exhibiting at least two chambers (2, 3), with the liquid fermented and pasteurized natural product being contained in the first chamber (2), and at least the probiotic microorganisms being contained in the second chamber (3)in a quantity that the natural product, after addition of the living probiotic microorganismus, contains 10⁵ to 10¹⁵ cells/ml of them, and wherein the second chamber (3) can be opened by a relative movement between the chambers (2, 3).

26. Container according to Claim 25, **characterised in that** one of the two chambers (2, 3) is pot-shaped and the other of the two chambers is formed by a closure with which the first chamber (2) is capable of being locked.

27. Container according to Claim 25 and/or 26, **characterised in that** the second chamber (3) exhibits an interlayer (4).

28. Container according to at least one of Claims 25 to 27, **characterised in that** the first chamber (2) and the second chamber (3) exhibit a screw thread.

29. Container according to at least one of Claims 25 to 28, **characterised in that** secondary plant substances, vitamins, minerals, fatty acids, polysaccharides, polyglucanes and/or extracts from fungi are further contained in the second chamber (3).

30. Use of the natural product according to at least one of Claims 1 to 13 for internal and external administration.

31. Use according to Claim 30 as foodstuff and food.

32. Use according to Claim 30 in a pack or in a compress.

## Revendications

1. Produit naturel liquide, fermenté et pasteurisé à base de fruits, légumes, légumineuses, plantes médicinales et/ou noix, contenant des micro-organismes probiotiques en une quantité de 10⁵ à 10¹⁵ cellules par ml, dans lequel la proportion de micro-organismes morts est présente dans le surnageant.

2. Produit naturel selon la revendication 1, **caractérisé en ce qu'**il contient les micro-organismes probiotiques en une quantité de 10⁶ à 10¹² cellules par ml.

3. Produit naturel selon la revendication 2, **caractérisé en ce qu'**il contient les micro-organismes probiotiques en une quantité de 10⁸ à 10¹⁰ cellules par ml.

4. Produit naturel selon l'une au moins des revendications 1 à 3, **caractérisé en ce qu'**il se compose d'un ferment ou d'un extrait fermenté de matières brutes.

5. Produit naturel selon l'une au moins des revendications 1 à 4, **caractérisé en ce que** les micro-organismes probiotiques sont choisis parmi des bactéries, des champignons, des levures et/ou des mélanges de ces organismes.

6. Produit naturel selon la revendication 5, **caractérisé en ce que** les micro-organismes probiotiques sont des bactéries.

7. Produit naturel selon l'une au moins des revendications 1 à 6, **caractérisé en ce qu'**il contient des micro-organismes probiotiques sous forme lyophilisée.

8. Produit naturel selon l'une au moins des revendications 5 à 7, **caractérisé en ce que** les bactéries probiotiques comprennent au moins une bactérie de l'ordre des *Lactobacillales.*

9. Produit naturel selon la revendication 8, **caractérisé en ce que** lesdites bactéries sont des bactéries de la famille des *Lactobacillacées.*

10. Produit naturel selon la revendication 9, **caractérisé en ce que** lesdites bactéries sont des bactéries du genre *Lactobacillus.*

11. Produit naturel selon la revendication 10, **caractérisé en ce que** lesdites bactéries sont des bactéries de l'espèce *Lactobacillus rhamnosus.*

12. Produit naturel selon l'une au moins des revendications 1 à 11, **caractérisé en ce que** les micro-organismes sont présents sous la forme d'un mélange de *Lactobacillales* avec d'autres micro-organismes probiotiques.

13. Produit naturel selon l'une au moins des revendications 1 à 12, **caractérisé en ce qu'**il contient en plus des substances végétales secondaires, des vitamines, des minéraux, des acides gras, des polysaccharides, des polyglucanes et/ou des extraits de champignons.

14. Procédé de préparation d'un produit naturel liquide fermenté selon l'une au moins des revendications 1 à 13 précédentes, comprenant les étapes consistant à :
(i) obvenir un produit naturel liquide, fermenté et pasteurisé à base de fruits, légumes, légumineuses, plantes médicinales et/ou noix ;
(ii) ajouter des micro-organismes probiotiques en une quantité de 10⁵ à 10¹⁵ cellules par ml audit produit naturel liquide, fermenté et pasteurisé ; et
(iii) mélanger les micro-organismes probiotiques avec le produit naturel liquide, fermenté et pasteurisé
immédiatement avant de le consommer.

15. Procédé selon la revendication 14, **caractérisé en ce que** les micro-organismes probiotiques sont ajoutés au produit naturel liquide fermenté en une quantité de 10⁶ à 10¹² cellules par ml.

16. Procédé selon la revendication 15, **caractérisé en ce que** les micro-organismes probiotiques sont ajoutés au produit naturel liquide fermenté en une quantité de 10⁸ à 10¹⁰ cellules par ml.

17. Procédé selon l'une au moins des revendications 14 à 16, **caractérisé en ce que** les micro-organismes probiotiques ajoutés sont des bactéries, des champignons, des levures et/ou des mélanges de ces organismes.

18. Procédé selon la revendication 17, **caractérisé en ce que** les micro-organismes probiotiques ajoutés sont des bactéries.

19. Procédé selon l'une au moins des revendications 14 à 18, **caractérisé en ce que** les micro-organismes probiotiques sont ajoutés sous forme lyophilisée.

20. Procédé selon l'une au moins des revendications 17 à 19, **caractérisé en ce que** les bactéries probiotiques ajoutées comprennent au moins une bactérie de l'ordre des *Lactobacillales.*

21. Procédé selon la revendication 20, **caractérisé en ce que** les bactéries ajoutées sont des bactéries de la famille des *Lactobacillacées.*

22. Procédé selon la revendication 21, **caractérisé en ce que** les bactéries ajoutées sont des bactéries du genre *Lactobacillus.*

23. Procédé selon la revendication 22, **caractérisé en ce que** les bactéries ajoutées sont des bactéries de l'espèce *Lactobacillus rhamnosus.*

24. Procédé selon l'une au moins des revendications 14 à 23, **caractérisé en ce qu'**au moins un micro-organisme de l'ordre des *Lactobacillales* est utilisé en mélange avec au moins un autre micro-organisme probiotique.

25. Récipient (1) destiné à conserver séparément un produit naturel liquide, fermenté et pasteurisé, et des micro-organismes probiotiques vivants afin de préparer un produit naturel selon l'une au moins des revendications 1 à 13 précédentes, lequel récipient (1) présente au moins deux compartiments (2, 3) dont le premier (2) contient le produit naturel liquide, fermenté et pasteurisé, et le deuxième (3) contient au moins les micro-organismes probiotiques en une quantité telle que, après leur ajout, le produit naturel contient lesdits micro-organismes probiotiques vivants en une quantité de 10⁵ à 10¹⁵ cellules par ml, et le deuxième compartiment (3) peut être ouvert par un mouvement relatif entre les compartiments (2, 3).

26. Récipient selon la revendication 25, **caractérisé en ce qu'**un des deux compartiments (2, 3) est en forme de pot et l'autre compartiment est formé par un bouchon au moyen duquel le premier compartiment (2) peut être fermé.

27. Récipient selon la revendication 25 et/ou la revendication 26, **caractérisé en ce que** le deuxième compartiment (3) présente une couche de séparation (4).

28. Récipient selon l'une au moins des revendications 25 à 27, **caractérisé en ce que** le premier compartiment (2) et le deuxième compartiment (3) présentent un filetage.

29. Récipient selon l'une au moins des revendications 25 à 28, **caractérisé en ce que** le deuxième compartiment (3) contient en plus des substances végétales secondaires, des vitamines, des minéraux, des acides gras, des polysaccharides, des polyglucanes et/ou des extraits de champignons.

30. Utilisation du produit naturel selon l'une au moins des revendications 1 à 13 pour une administration interne et externe.

31. Utilisation selon la revendication 30 comme aliment et nutriment.

32. Utilisation selon la revendication 30 dans un bandage ou une compresse.
